# EUROPEAN PATENT APPLICATION

(11) **EP 4 438 740 A1**
(43) Date of publication of application: **02.10.2024**
(21) Application number: 22897157.8
(22) Date of filing: 04.07.2022
(51) Int. Cl.: C12Q 1/6844

(54) **METHOD FOR PREPARING CONSTANT-TEMPERATURE AMPLIFICATION MIXED ENZYME SYSTEM**

(30) Priority: 23.11.2021 CN 202111395102
(71) Applicant: Daan Gene Co., Ltd., Guangzhou, Guangdong 510665 (CN)
(72) Inventor: JIANG, Xiwen, Guangzhou, Guangdong 510665 (CN); LIAN, Xianlan, Guangzhou, Guangdong 510665 (CN); XIE, Xiaocheng, Guangzhou, Guangdong 510665 (CN); LU, Xuelan, Guangzhou, Guangdong 510665 (CN); XU, Haojian, Guangzhou, Guangdong 510665 (CN)
(74) Representative: Diehl & Partner
(86) International application number: PCT/CN2022/103697
(87) International publication number: WO 2023/093061

(57) **Abstract**

Provided in the present invention is a method for preparing a constant-temperature amplification mixed enzyme system. Specifically, disclosed in the present invention is a method comprising: removing the vast majority of glycerol from a prepared glycerol-containing RPA enzyme system by means of dialysis, and then concentrating the enzyme system containing trace glycerol by using an ultrafiltration method. Experiments show that the amplification performance of an RPA mixed enzyme system prepared by means of such method is significantly improved.

## Description

### Technical field

The invention belongs to the field of biotechnology. In particular, the invention relates to a method for preparing a constant-temperature amplification mixed enzyme system.

### Background

Freeze-drying technology (Lyophilization technology) is a drying method that freezes aqueous materials below the freezing point, turns water into ice, and then turns ice into steam under a higher vacuum to remove it. The enzyme lyophilized powder made by freeze-drying technology can avoid repeated freeze-thaw during low-temperature storage, which is a good and universal method for enzyme preservation.

Constant-temperature amplification technology is a technology that uses constant temperature for reaction in the process of nucleic acid amplification. Because it does not need the qPCR instrument necessary for qPCR technology, it can greatly reduce the cost of instrument. It can also be made into smaller volume for carry without a heating instrument with precise temperature control, which greatly facilitates the application of constant-temperature amplification technology. Recombinase polymerase amplification (RPA) is a kind of constant-temperature amplification technology, which is characteristic of high efficiency, high sensitivity and strong specificity so that it is gradually valued in the field of nucleic acid detection.

RPA reaction usually uses 5-8 single enzymes to start a reaction. Experiments show that these 5-8 single enzymes are unstable when stored at -20°C after mixed into a complete RPA enzyme system. Therefore, the RPA enzyme system is lyophilized to realize the long-term storage of the formulated enzyme system at low temperature. However, for the existing freeze-drying methods for preparing RPA mixed enzyme system, there exists the problems of large enzyme activity loss and low reaction efficiency.

Therefore, those skilled in the art are working to develop a preparation process that can avoid loss of enzyme activity in RPA enzyme system.

### Summary of invention

The invention aims to provide a method capable of stably preparing a constant-temperature amplification enzyme system.

In the first aspect of the invention, there is provided a method for preparing a constant-temperature amplification mixed enzyme system, which comprises the steps of:
(1) providing single enzymes required for the constant-temperature amplification mixed enzyme system, wherein each single enzyme is independently stored in a corresponding preservation solution, and the glycerol content of each preservation solution is about 20% - 70% (w/w);
(2) mixing the single enzymes provided in step (1) to prepare a mixed enzyme solution;
(3) dialyzing the mixed enzyme solution obtained in step (2); and optionally,
(4) concentrating the dialyzed mixed enzyme solution.

In another preferred example, the constant-temperature amplification mixed enzyme system is an RPA mixed enzyme system.

In another preferred example, the method further comprises the steps of:
(5) lyophilizing the mixed enzyme solution after dialysis or concentration.

In another preferred example, the single enzyme required for the constant-temperature amplification mixed enzyme system includes: a recombinase, a single-stranded binding protein, and a DNA polymerase.

In another preferred example, the single enzyme required for the constant-temperature amplification mixed enzyme system also includes: a loading protein.

In another preferred example, the single enzyme required for the constant-temperature amplification mixed enzyme system also includes: an exonuclease, and/or a creatine kinase.

In another preferred example, the single enzyme required for the constant-temperature amplification mixed enzyme system also includes one or more components selected from the group consisting of: a reverse transcriptase, RNasin, and an inorganic pyrophosphatase.

In another preferred example, the loading protein is loading protein UvsY.

In another preferred example, the exonuclease is Exonuclease III (EXO).

In another preferred example, the recombinant enzyme is recombinant enzyme UvsX.

In another preferred example, the single-stranded binding protein is single-stranded binding protein gp32.

In another preferred example, the DNA polymerase is DNA polymerase B su.

In another preferred example, the reverse transcriptase is reverse transcriptase MMLV.

In another preferred example, in step (3), the dialysate used for dialysis includes: 20-80mM Tris, 200-400mM NaCl, 0.05-0.2mM EDTA, 0.5-2 DTT, and the pH of the dialysate is 6.5-7.5; preferably, the dialysate used for dialysis includes 50mM Tris, 300mM NaCl, 0.1mM EDTA, 1mM DTT, and the pH is 7.0.

In another preferred example, in step (3), the dialysis bag used for dialysis is a 5-10KD dialysis bag.

In another preferred example, in step (3), the dialysis temperature is about 0-10°C, preferably 0-4°C; the dialysis time is 5-24 hours, preferably 10-20 hours.

In another preferred example, in step (3), the volume of dialysate used for dialysis is 50-200 times, preferably 100 times that of the mixed enzyme solution.

In another preferred example, in step (4), ultrafiltration tube is used to concentrate the mixed enzyme solution after dialysis.

In the second aspect of the present invention, there is provided a kit, which comprises a constant-temperature amplification mixed enzyme system prepared using the method described in the first aspect of the present invention.

In another preferred example, the kit also includes specific primers and/or probes.

In another preferred example, the kit includes a first container with a constant-temperature amplification mixed enzyme system prepared using the method described in the first aspect of the present invention.

In another preferred example, the kit also includes a second container with one or more components selected from the group consisting of: creatine phosphate, Tris-HCl, ATP, DTT, dNTPs, potassium acetate, and PEG.

In another preferred example, the kit also includes a third container with magnesium acetate.

In another preferred example, the kit also includes a fourth container with water or buffer solution.

It should be understood that, within the scope of the present invention, the above technical features of the present invention and the technical features specifically described below (as in Examples) can be combined with each other to form a new or preferred technical solution. Due to the limitation of space, it will not be repeated one by one herein.

### Description of drawings

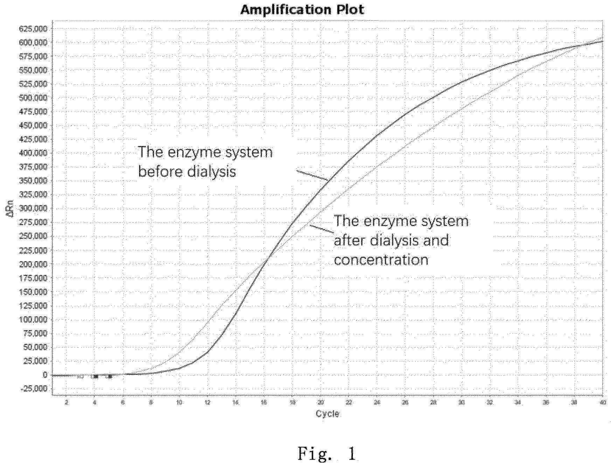

Fig. 1 shows the detection results in Example 1.

### Detailed description of invention

Through extensive and intensive research, the inventor accidentally found that the configured constant-temperature amplification enzyme system containing glycerol was dialyzed to remove vast majority of glycerol, and then the enzyme system containing trace glycerol was concentrated by using an ultrafiltration method. Experiments show that the performance of the constant-temperature amplification enzyme system containing trace glycerol prepared by this method is better than that of the enzyme system containing glycerol. On this basis, the present invention has been completed.

Before describing the present invention, it should be understood that the present invention is not limited to the specific methods and experimental conditions described, because such methods and conditions may vary. It should also be understood that the terms used herein are intended only to describe specific embodiments and are not intended to be restrictive, and the scope of the invention will be limited only by the appended claims.

Unless otherwise defined, all technical and scientific terms used herein have the same meaning as generally understood by those skilled in the art to which the present invention belongs. As used herein, when referring to a specific enumerated value, the term **"about"** means that the value can vary by no more than 1% from the enumerated value. For example, as used herein, the expression "about 100" includes all values between 99 and 101 (for example, 99.1, 99.2, 99.3, 99.4, etc.).

Although any method and material similar or equivalent to those described in the invention can be used in the implementation or testing of the invention, the preferred methods and materials are exemplified herein.

Enzymes usually need to be stored at lower temperature for a long-term preservation. Because repeated freezing and thawing will affect enzyme activity, 50% glycerol is usually added to the storage solution to ensure that the enzyme is stored in liquid state at -20°C. However, some enzymes are still unstable when stored in liquid state at -20°C, or the addition of glycerol needs to be avoided for the enzyme itself during the reaction process. In these conditions, use of 50% glycerol storage solution is not conducive to the preservation of enzyme.

The inventor found that the storage solution of a constant-temperature amplification (such as RPA) enzyme system has poor stability of single enzyme when stored in frozen state, and the presence of glycerol will reduce the solidification temperature of enzyme system, resulting in poor freezing effect of the enzyme system during lyophilization. Glycerol can be removed in the preparation process of each single enzyme of RPA to obtain each single enzymes without glycerol in the storage solution, but the single enzymes with glycerol removed cannot be stored at low temperature of -20°C, and each single enzyme needs to be subjected to quality test and concentration determination in advance before being formulated into a complete RPA enzyme system, which leads to the formulated single enzymes to be stored above 0°C for a period of time, and the storage process above 0°C will cause damage to enzyme activity, which reduces the effect of the formulated complete RPA enzyme system.

The present invention removes a vast majority of glycerol from the formulated RPA enzyme system containing glycerol by dialysis, and then concentrates the enzyme system containing trace glycerol by ultrafiltration. Experiments show that the performance of the RPA enzyme system containing trace glycerol prepared by this method is better than that of enzyme system containing glycerol.

### Recombinase polymerase amplification

Recombinase polymerase amplification (RPA) is a constant-temperature amplification technology. Disclosure of RPA methods, including US patent document US 7,270,981; US 7,399,590; US 7,666,598; US 7,435,561; US patent publication US 2009/0029421; International application WO 2010/141940.

The key enzymes of RPA system include recombinase that can bind single-stranded nucleic acids (oligonucleotide primers), single-stranded DNA binding protein (SSB) and strand displacement DNA polymerase. The protein DNA complex formed by the combination of recombinase and primers can search for homologous sequences in double-stranded DNA. Once the primers locate the homologous sequence, strand exchange reaction will occur to form and start DNA synthesis, and the target region on the template will be exponentially amplified. The replaced DNA strand binds to SSB to prevent further replacement.

At the start of RPA reaction, the recombinase can pair oligonucleotide primers homologous to the double-stranded DNA template. When there is a nucleotide template, two or more sequence specific primers (such as gene specific) are used to start the exponential amplification reaction. The reaction progresses rapidly. The result of specific amplification of double-stranded DNA template sequence is that the DNA template is amplified from only a few copies to a detectable level within a few minutes.

The invention adds reverse transcriptase, RNasin and inorganic pyrophosphatase on the basis of real-time fluorescence RPA technology. Reverse transcriptase and RNasin are simultaneously used in the reaction system to start and maintain the normal progress of RNA template amplification reaction. The addition of inorganic pyrophosphatase significantly improves the reaction efficiency.

### Recombinases

The recombinase (recombinant protein) used for RPA reaction can be from prokaryotes, viruses or eukaryotes. Typical recombinases include UvsX and RecA (such as RecA protein or UvsX protein obtained from any species), and their fragments or mutants, or any combination thereof.

RecA and UvsX proteins can be obtained from any species, and RecA and UvsX fragments or mutant proteins can also be generated with appropriate RecA and UvsX proteins, nucleotide sequences, and molecular biology techniques (see the formation of UvsX mutants as described in U.S. Patent No. 8,071,308). Typical UvsX proteins include those derived from myoviridae phages, such as T4, T2, T6, Rb69, Aehl, KVP40, Acinetobacter phage 133, Aeromonas phage 65, cyanophage P-SSM2, cyanophage PSSM4, cyanophage S-PM2, Rb14, Rb32, Aeromonas phage 25, Vibrio bacteriophage NT-1, phi-1, Rb16, Rb43, phage 31, phage 44RR2.8t, Rb49, phage Rb3, and phage LZ2. Other typical recombinase proteins include archaeal RADA and RADB proteins and eukaryotic (such as plants, mammals and fungi) Rad51 proteins (such as RAD51, RAD51B, RAD51C, RAD51D, DMC1, XRCC2, XRCC3, and recA) (see E.g. Lin et al., Proc.Natl.Acad.Sci.U.S.A.103:10328-10333,2006).

In a preferred embodiment of the present invention, the recombinase is UvsX. See NCBI serial number NP_049656.2 for the amino acid sequence of typical UvsX.

### Single-stranded binding proteins

Single-stranded binding proteins (ssDNA binding proteins) can be used to stabilize nucleotides. One or more ssDNA binding proteins can be derived from or obtained from various species, such as prokaryotes, viruses or eukaryotes. Typical single-stranded DNA binding proteins include but are not limited to *E. coli* SSB and those single-stranded DNA binding proteins derived from myoviral phages, such as T4, T2, T6, Rb69, Aehl, KVP40, Acinetobacter phage 133, Aeromonas phage 65, cyanobacteria phage, Vibrio phage, etc.

In the present invention, the single-stranded binding protein is preferably the single-stranded binding protein gp32. gp32 protein plays a key role in DNA replication, recombination and repair of T4 phage. The most important reason is that gp32 protein has the property of tightly binding to single-stranded DNA. See NCBI serial number NP_049854 for the amino acid sequence of typical gp32 protein.

### DNA polymerases

DNA polymerases can be eukaryotic or prokaryotic polymerases. Examples of eukaryotic polymerases include pol-alpha, pol-beta, pol-delta, pol-epsilon, and their mutants or fragments, or combinations thereof. Examples of prokaryotic polymerases include *E. coli* DNA polymerase I (such as Klenow fragment), phage T4 gp43 DNA polymerase, large fragment of Bacillus stearothermophilus polymerase I, Phi-29 DNA polymerase, T7 DNA polymerase, Bacillus subtilis Pol I, Staphylococcus aureus Pol I, *E. coli* DNA polymerase I, *E. coli* DNA polymerase II, *E. coli* DNA polymerase III, *E. coli* DNA polymerase IV, *E. coli* DNA polymerase V, and their mutants or fragments, or combinations thereof.

In the present invention, the DNA polymerase is preferably BSU DNA polymerase. Generally, Bsu DNA polymerase (a large fragment Bsu DNA polymerase, Bsu DNA Polymerase Large Fragment) is derived from Bacillus subtilis by truncating the first 296 codons of the Bacillus subtilis DNA polymerase I gene. This fragment retains 5'-3' polymerase activity of Bsu DNA polymerase, but lacks 5'-3' exonuclease activity. At the same time, this large fragment itself lacks 3'-5' exonuclease activity. See NCBI serial number AAR00931.1 for the amino acid sequence of typical Bsu DNA polymerase.

### Loading proteins

Loading proteins can be derived from prokaryotes, viruses or eukaryotes. Typical loading proteins include *E. coli* RecO, *E. coli* RecR, UvsY, and their mutants or fragments, or their combinations. Typical Uvsy proteins include those derived from myoviral phages such as T4, T2, T6, Rb69, Aehl, KVP40, Acinetobacter phage 133, Aeromonas phage 65, cyanobacterial P-SSM2, cyanobacterial PSSM4, cyanobacterial S-PM2, Rb14, Rb32, Aeromonas phage 25, arcobacterial phage nt-1, phi-1, Rb16, Rb43, phage 31, phage 44RR2.8t, Rb49, phage Rb3, and phage LZ2.

In the present invention, the loading protein is preferably the loading protein UvsY. See NCBI serial number NP_049799.2 for the amino acid sequence of the typical loading protein UvsY.

### Exonucleases

Exonucleases refer to the general name of several subclasses of enzymes in the hydrolase class (EC3.1.11.13.14.15.16), which can catalyze the hydrolysis of phosphodiester bonds at the ends of nucleic acid chains and cut nucleotides one by one. According to substrate specificity, they can be divided into DNA exonucleases, RNA exonucleases and exonucleases that can act on both DNA and RNA.

In a preferred embodiment of the present invention, the exonuclease of the invention is Exonuclease III. See NCBI serial number WP_000673937.1 for the amino acid sequence of typical Exonuclease III.

### Creatine Kinase

Creatine kinase (CK) is an important kinase directly related to intracellular energy transport, muscle contraction and ATP regeneration. It reversibly catalyzes the transphosphorylation reaction between creatine and ATP.

Creatine kinase can be selected from rabbit muscle kinase, carp muscle kinase, such as carp muscle kinase M1 type (M1-CK). See NCBI serial number NP_001075708.1 for the amino acid sequence of typical creatine kinase.

### Reverse transcriptase

Reverse transcriptase is also known as RNA dependent DNA polymerase. The enzyme uses RNA as template, dNTP as substrate, tRNA (mainly tryptophan tRNA) as primer, and synthesizes a DNA single strand complementary to the RNA template in the direction of 5'-3'on the 3'-OH end of tRNA according to the principle of base pairing.The DNA single strand is called complementary DNA (cDNA).

Reverse transcriptase can be used for synthesizing first strand cDNA, making cDNA probe, RNA transcription, sequencing and reverse transcription reaction of RNA. Reverse transcriptases commonly used in this field include murine leukemia virus (M-MLV) reverse transcriptase and avian myeloblastoma virus (AMV) reverse transcriptase. See NCBI serial number AAA66622.1 for the amino acid sequence of typical creatine kinase.

### RNasin

RNasin is a protein inhibitor of RNase. It can be extracted from rat liver or human blastoderm or prepared by gene recombination. RNasin is a non-competitive inhibitor of RNases, which can bind to a variety of RNases to inactivate them. See NCBI serial number P13489.2 for the amino acid sequence of typical RNasin.

### Inorganic pyrophosphatase

Inorganic pyrophosphatase (IPP) catalyzes the hydrolysis of inorganic pyrophosphate to produce orthophosphate. Inorganic pyrophosphatase can be prepared by recombinant *E. coli* strain or yeast strain carrying inorganic pyrophosphatase gene. See NCBI serial number WP_000055072.1 for the amino acid sequence of typical inorganic pyrophosphatase.

### RT-RPA reaction system and detection method

In a preferred embodiment, the RT-RPA reaction system of the present invention comprises:
Recombinant protein UvsX, loading protein UvsY, single-stranded binding protein gp32, Exonuclease III, Bsu DNA polymerase, creatine kinase CK, reverse transcriptase (MMLV), RNasin, IPP enzyme, phosphocreatine (PC), Tris-HCl (pH7.5), ATP, dithiothreitol (DTT), dNTPs, potassium acetate (KOAc), magnesium acetate, PEG (such as PEG35000).

In another preferred embodiment, the content of each component in the RT-RPA reaction system is as follows:

| Component | Content |
|---|---|
| Recombinant protein UvsX | 100-1000ng/µL |
| Loading protein UvsY | 100-500ngµL |
| Single-stranded binding protein gp32 | 500-2000ng/µL |
| Exonuclease III | 1-10ng/µL |
| Bsu DNA polymerase | 5-15ng/µL |
| Creatine kinase CK | 0.1-5ng/µL |
| Reverse transcriptase (MMLV) | 1-10U/µL |
| RNasin | 1-50U/µL |
| IPP enzyme | 0.1-5U/µL |
| Phosphocreatine (PC) | 50-500mM |
| Tris-HCl (pH7.5) | 5-20mM |
| ATP | 5-20mM |

| | |
|---|---|
| Dithiothreitol (DTT) | 0.1-5mM |
| dNTPs | 50-500µM |
| Potassium acetate (KOAc) | 10-100mM |
| Magnesium acetate | 5-25 mM |
| PEG35000 | 0.5-10% (w/w) |

Preferably, in a RT-RPA reaction system, the concentration of upstream primer is 10-200 nM, the downstream primer is 100 nM, the probe is 50-300 nM, and the RNA template is 1-10 µL. Preferably, the total volume of RT-qRPA system is 15-50 µL. The rest is supplemented with double distilled water.

Reaction procedure:
Constant temperature at 20-45°C and sustained reaction for 10-40 minutes.

The reaction endpoint was monitored by agarose gel electrophoresis, SYBR Green I, or specific probes. When monitored with SYBR Green I, Sybr Green I with final concentration of 0.3-0.5x was added into the reaction system. The reaction time can be 20-40min, and the fluorescence can be read every 30s. ABI7500, FTC-3000, Bio-Rad CFX MiniOpticon System, GenDx thermostatic fluorescence detector GS8 etc. can be used as instruments for detection.

If a specific probe is used to detect the amplification system, the final concentration of fluorescently labeled probe is 10-500nM. ABI QuantStudio 5, ABI7500, FTC-3000, Bio-Rad CFX MiniOpticon System, GenDx thermostatic fluorescence detector GS8 etc. were used for fluorescence detection.

### The main advantages of the present invention are:

The method for preparing RPA mixed enzyme system provided by the invention can significantly improve the amplification efficiency of freeze-dried samples of RPA mixed enzyme system.

The method of the invention can save the cost of additional preparation of glycerol free RPA enzyme systemwithout additional production of glycerol free RPA single enzyme, which improves the efficiency of preparing mixed enzyme system. At the same time, it is verified that the method can also improve the performance of the reaction system.

The present invention will be further described in detail below in combination with specific examples. It should be understood that these examples are only used to illustrate the invention and not to limit the scope of the invention. The experimental methods for which specific conditions are not indicated in the following examples are generally in accordance with conventional conditions such as those described in Molecular Cloning: A Laboratory Manual, by Sambrook. J et al.(ed.), U.S.A. (Translated by Huang Peitang et al., Beijing: Science Press, 2002), or in accordance with the conditions recommended by the manufacturer. Unless otherwise indicated, all percentage and parts are calculated by weight. Unless otherwise stated, the experimental materials and reagents used in the following examples are available from commercially available sources.

### Example 1

The single enzymes required for RPA enzyme system were stored separately in preservation solution, which was glycerol aqueous solution with a glycerol content of 50% (w/w). The single enzymes are: recombinant protein UvsX, loading protein UvsY, single-stranded binding protein gp32, Exonuclease III, Bsu DNA polymerase, creatine kinase CK, reverse transcriptase (MMLV), RNasin, and IPP enzyme.

The RPA mixed enzyme system (50ml) was prepared by mixing 9 kinds of single enzymes required for the RPA enzyme system according to specific ratio. Since 8 kinds of single enzymes were stored in preservation solution containing 50% glycerol, thus the formulated storage solution of RPA enzyme system also contained 50% glycerol.

The dialysate of glycerol-removed enzyme system was prepared with the following formula: 50mM Tris, 300mM NaCl, 0.1mM EDTA, 1mM DTT, pH 7.0.

The glycerol containing RPA enzyme system was put into a 10KD dialysis bag. After sealing the two ends of the dialysis bag, it was put into 500mL dialysate of glycerol-removed enzyme system, and then the dialysate was placed at 4°C for more than 16 hours.

The RPA enzyme system was removed from the dialysis bag after dialysis. Dialysis of the enzyme system containing glycerol in the dialysate without glycerol will result in an increase in the volume of the enzyme system. Therefore, the enzyme system after dialysis was put into a 3KD ultrafiltration tube, the ultrafiltration tube was put into a centrifuge at a set speed of 5000rpm for continuous 30 minutes to concentrate the enzyme system. The volume of finally obtained mixed enzyme system containing trace glycerol is the same as that of mixed enzyme system before dialysis.

The enzyme content in the RT-RPA reaction system used in this example is as follows:

| Component | Content |
|---|---|
| Recombinant protein UvsX | 500ng/µL |
| Loading protein UvsY | 228ng µL |
| Single-stranded binding protein gp32 | 919ng/µL |
| Exonuclease III | 3ng/µL |
| Bsu DNA polymerase | 9.5ng/µL |
| Creatine kinase CK | 1ng/µL |
| Reverse transcriptase (MMLV) | 5U/µL |
| RNasin | 20U/µL |
| IPP enzyme | 1U/µL |

The mixed enzyme system was prepared according to the above ratio.

The RPA reaction solution used in this example is as follows:

| Components of RPA basic reaction solution | Concentrations of components of RPA reaction solution |
|---|---|
| Phosphocreatine (PC) | 1mM |
| Tris-HCl (pH7.5) | 10mM |
| ATP | 10mM |
| Dithiothreitol (DTT) | 2.7mM |
| dNTPs | 25 µ M |
| Potassium acetate (KOAc) | 100mM |
| Magnesium acetate | 15 mM |
| PEG35000 | 3% |

According to what is shown in the table, RPA basic reaction solution was prepared and 100nM reaction upstream primer, 100nM reaction downstream primer, 150nM reaction probe and 8 µL RNA templates with different concentrations for the reaction were added. Then a complete RT-qRPA system was formed, with a total system of 20 µL. The rest of 20 µL was supplemented with double distilled water to 20 µL.

### Reaction procedure:

Constant temperature at 42°C for continuous 20 minutes, wherein the detection channel was FAM, and the fluorescence signal was detected every 30 seconds. The detection was performed on ABI QuantStudio 5 quantitative PCR instrument.
The target of detection is apoB gene: 
Cagtgtatctggaaagcctacaggacaccaaaataaccttaatcatcaattggttacaggaggctttaagttcag
catctttggctcacatgaaggccaaattccgagagactctagaagatacacgagaccgaatgtatcaaatggacattcag
caggaacttcaacgatacctgtctctggtaggccaggtttatagcacacttgtcacctacatttctgattggtggactcttgc
tgctaagaaccttactgactttgcagagcaatattctatccaagattgggctaaacgtatgaaagca (SEQ ID
NO.1)
Upstream primer: CAGTGTATCTGGAAAGCCTACAGGACACCAAAA (SEQ ID NO.2)
Downstream primer: TGCTTTCATACGTTTAGCCCAATCTTGGATAG (SEQ ID NO.3)
Probe: attcagcaggaacttcaacgatacctgtctcTg-THF-Taggccaggtttatagca[C3-spacer] (SEQ ID NO.4)
The 32nd base is T, which is modified into a base with FAM fluorescent group;
A THF (tetrahydrofuran) site was added between the 33rd base and the 34th base;
The 34th base is T, which is modified into a base with BHQ1 quenching group;
C3**-spacer was added at 3' end**

The apoB gene shown above was cloned into a plasmid, then T7 in vitro transcription promoter TAATACGACTCACTATAGG (SEQ ID No.5) was added to 5' end of the template by PCR. Then transcription was performed through the recognition of the promoter by T7 RNA polymerase to obtain the corresponding apoB RNA template. Then, DNase was added to digest the DNA template, and finally inactivated by heating at 80°C for 2min.

The detection results are shown in Fig. 1. The amplification curve represents the test results of the test group using the mixed enzyme system lyophilized product without dialysis treatment and the mixed enzyme system lyophilized product after dialysis and concentration treatment, respectively. The CT value of the enzyme system test group after dialysis and concentration is 8.697, and the CT value of the enzyme system test group before dialysis treatment is 10.749. The results show that this preparation method not only reduces the glycerol content of the enzyme system from 50% to less than 1%, but also significantly improves the amplification efficiency of the reaction system.

All the documents cited herein are incorporated into the invention as reference, as if each of them is individually incorporated. In addition, it should be understood that after reading the above teaching content of the invention, those skilled in the art could make various changes or modifications to the invention, and these equivalent forms would still be in the scope of the invention defined by the appended claims of the application.

## Claims

1. A method for preparing a constant-temperature amplification mixed enzyme system, which is **characterized in that** the method comprises the steps of:
(1) providing single enzymes required for the constant-temperature amplification mixed enzyme system, and each single enzyme is independently stored in corresponding preservation solution, and the glycerol content in each preservation solution is about 20% - 70% (w/w);
(2) mixing the single enzymes provided in step (1) to prepare a mixed enzyme solution;
(3) dialyzing the mixed enzyme solution obtained in step (2); and
optionally,
(4) concentrating the dialyzed mixed enzyme solution.

2. The method according to claim 1, which is **characterized in that** the method further comprises the steps of:
(5) lyophilizing the mixed enzyme solution after dialysis or concentration.

3. The method according to claim 1, which is **characterized in that** the single enzymes required for the constant-temperature amplification mixed enzyme system include: a recombinase, a single-stranded binding protein, and a DNA polymerase.

4. The method according to claim 1, which is **characterized in that** the single enzymes required for the constant-temperature amplification mixed enzyme system also include: a loading protein, an exonuclease, and/or a creatine kinase.

5. The method according to claim 1, which is **characterized in that** the single enzymes required for the constant-temperature amplification mixed enzyme system also include one or more components selected from the group consisting of: reverse transcriptase, RNasin, and inorganic pyrophosphatase.

6. The method according to claim 1, which is **characterized in that** in step (3), the dialysate used for dialysis includes: 20-80mM Tris, 200-400mM NaCl, 0.05-0.2mM EDTA, 0.5-2 DTT, and the pH of the dialysate is 6.5-7.5.

7. The method according to claim 1, which is **characterized in that** in step (3), the dialysis bag used for dialysis is a 5-10KD dialysis bag.

8. The method according to claim 1, which is **characterized in that** in step (3), the dialysis temperature is about 0-10°C, preferably 0-4°C; the dialysis time is 5-24 hours, preferably 10-20 hours.

9. The method according to claim 1, which is **characterized in that** in step (4), an ultrafiltration tube is used to concentrate the dialyzed mixed enzyme solution.

10. A kit, which is **characterized in that** the kit comprises an RPA mixed enzyme system prepared using the method according to claim 1.
